# DEMANDE DE BREVET EUROPEEN

(11) **EP 4 659 719 A1**
(43) Date de publication de la demande: **10.12.2025**
(21) Numéro de dépôt: 24179947.7
(22) Date de dépôt: 04.06.2024
(51) Int. Cl.: A61F 5/01, A61F 5/058

(54) **DISPOSITIF MÉDICAL POUR MEMBRES OU ARTICULATIONS IMPRIMÉ EN 3D ET SON SYSTÈME DE FERMETURE PAR AIMANT AINSI QUE SON PROCÉDÉ DE FABRICATION**

(71) Demandeur: Skelenn, 29200 Brest (FR)
(72) Inventeur: DI FRANCIA, Rémi, 29200 Brest (FR); DAOULAS, Thomas, 29200 Brest (FR); TANNER, Jean-Loup, 29200 Brest (FR)
(74) Mandataire: Santarelli

(57) **Abrégé**

Dispositif médical (100,200,300,400) pour immobiliser un membre ou une articulation, ledit dispositif comportant au moins deux demis-coques complémentaires (10a-b,20a-b,30a-b,40a-b) de forme sensiblement alvéolaire, lesdites demis-coques comprenant chacune au moins deux surfaces de contact (101a-b,102a-b,201a-b,202a-b,203a-b,301a-b,302a-b,303a-b,401a-b,402a-b), un système de fermeture (12,22,32,42), le système de fermeture (12,22,32,42) étant positionné dans des logements (103a-b,204a-b,304a-b,403a-b) des surfaces de contact (101a-b,102a-b,201a-b,202a-b,203a-b,301a-b,302a-b,303a-b,401a-b,402a-b), ledit système de fermeture étant magnétique, ledit système de fermeture étant invisible lorsque le dispositif médical (100,200,300,400) est assemblé.

## Description

### DOMAINE TECHNIQUE

La présente invention appartient au domaine de l'orthopédie et des dispositifs médicaux utilisés pour soutenir, stabiliser ou corriger des problèmes musculo-squelettiques.

La présente invention concerne plus particulièrement une attelle imprimée en 3D et son système de fermeture par aimant afin de faciliter son installation et son maintien sur un membre soigné.

La présente invention trouve une application directe en médecine orthopédique ainsi que pour les soins de traumatismes et d'articulation nécessitant d'être au repos.

### ÉTAT DE L'ART

On retrouve l'utilisation des premières attelles à l'époque de l'Antiquité pour immobiliser des fractures osseuses et des entorses. Ces attelles étaient alors fabriquées en matériaux naturels (bois, cuir, etc.).

Par la suite et grâce au développement ainsi qu'aux progrès de la médecine, les attelles sont de plus en plus perfectionnées.

Actuellement, les immobilisations sont faites soit en plâtre de Paris, soit en résine. L'utilisation de ces matières présentent de nombreux inconvénients.

Tout d'abord ces immobilisations sont lourdes et ne sont pas compatibles avec l'eau. De plus, elles ne permettent pas une aération optimale de la surface de la peau qui en est recouverte, les rendant malodorantes.

Par ailleurs, ces immobilisations sont souvent inconfortables pour le patient.

Avec l'avènement de la fabrication additive, notamment grâce aux imprimantes 3D basées sur la technologie du dépôt de fil fondu (en anglais *Fused Déposition Modelling, FDM*)*,* de nouvelles solutions d'attelles, qui plus est sur mesure, voient le jour et sans faire de compromis sur l'efficacité de la contention.

Le document US2018357348A1 décrit un dispositif d'immobilisation post-traumatique et un procédé de fabrication associé. Le dispositif d'immobilisation post-traumatique a une forme architecturale arrondie, formée par au moins deux parties rigides complémentaires, en forme de filet. Les parties rigides définissent des cavités concaves respectives et comprennent des extrémités de connexion incurvées, ainsi que des attaches pourvues de parties surélevées pour l'accouplement de joints toriques élastiques qui serrent les parties rigides l'une contre l'autre. Bien que le dispositif décrit dans le document US2018357348A1 permette d'améliorer le confort des patients, pour certains d'entre eux, l'accouplement de joints toriques ne peut se faire sans l'aide d'une tierce personne. Par ailleurs, les joints toriques élastiques pouvant de dégrader dans le temps, il peut être nécessaire de devoir les remplacer.

Le document EP2726029A1 décrit quant à lui un dispositif de maintien qui relie une première partie du corps à une deuxième partie du corps et comprend une première face et une deuxième face reliées par au moins un manchon, destiné à entourer la première partie du corps, et par une partie d'appui, destinée à être en appui contre la deuxième partie du corps, ledit manchon et ladite partie d'appui étant reliés par des moyens de blocage de la fermeture de l'articulation. Afin de fixer le dispositif de maintien, des aimants, bandes autoagrippantes ou boutons poussoirs peuvent être utilisé.

Le document WO9420049A1 décrit une attelle thérapeutique à traction dynamique pour le traitement de l'arthrose de l'articulation interphalangienne d'un doigt. Cette attelle comprend deux moyens qui s'accrochent aux phalanges et qui se repoussent par l'effet d'un champ magnétique ou d'un ressort. Dans ce document, les aimants sont utilisés afin de repousser les parties de l'attelle thérapeutique et non pour les attirer.

Afin d'améliorer le confort ainsi que l'acceptation du port d'attelles, il est donc nécessaire de continuer leurs développements afin de faciliter leurs usages, tout en garantissant le maintien nécessaire à la consolidation d'une fracture par exemple.

### PRÉSENTATION DE L'INVENTION

La présente invention vise à remédier, au moins en partie, aux inconvénients précités, en menant en outre à d'autres avantages.

La présente invention propose alors un dispositif médical réalisé au moyen du procédé de fabrication additive, ledit dispositif comprenant un système de fermeture par aimant permettant d'assembler deux demis-coques entre elles, formant ainsi une attelle complète.

A cet effet, la présente invention vise un dispositif médical pour immobiliser un membre ou une articulation, ledit dispositif comportant au moins deux demis-coques complémentaires de forme sensiblement alvéolaire, lesdites demis-coques comprenant chacune au moins deux surfaces de contact, un système de fermeture. Le dispositif est remarquable en ce que le système de fermeture est positionné dans des logements des surfaces de contact, ledit système de fermeture étant magnétique, ledit système de fermeture étant invisible lorsque le dispositif médical est assemblé.

Avantageusement, le système de fermeture comporte une pluralité d'aimant, chaque aimant étant positionné un seul à la fois dans le logement qui lui est dédié. De façon avantageuse, le système de fermeture comporte une pluralité de paires « aimant-cylindre métallique ».

Selon une caractéristique particulière, les logements du système de fermeture sont disposés régulièrement sur les surfaces de contact.

Selon une autre caractéristique particulière, les logements du système de fermeture sont disposés irrégulièrement sur les surfaces de contact. Avantageusement, les logements du système de fermeture sont disposés sur les surfaces de contact proportionnellement par rapport aux efforts mécaniques qui sont exercés sur ledit dispositif.

De façon avantageuse, les aimants ont une adhérence magnétique identique. Selon une caractéristique particulière, les aimants ont une adhérence magnétique différente.

Selon une autre caractéristique particulière, l'adhérence magnétique des aimants tient compte des efforts de traction qui sont exercés au niveau des emplacement desdits aimants sur ledit dispositif.

La présente invention a également pour objet un procédé de fabrication d'un dispositif médical tel que décrit précédemment, ledit procédé étant remarquable en ce qu'il comprend les étapes suivantes :
- de numérisation et de modélisation en trois dimensions (3D) pour obtenir un modèle 3D d'un membre ou d'une l'articulation à immobiliser ;
- d'importation du modèle 3D dans le logiciel de conception assistée par ordinateur (CAO) et de centrage du modèle 3D du membre ou de l'articulation à immobiliser dans un référentiel du logiciel de CAO ;
- de rectificatif de limites du modèle 3D du membre ou de l'articulation à immobiliser ;
- de génération d'un maillage basse résolution du modèle 3D obtenu à l'étape précédente ;
- de génération d'un maillage final en appliquant différents modificateurs sur le maillage basse résolution obtenu à l'étape précédente ;
- de génération et de positionnement des logements ;
- de séparation d'une structure du dispositif médical obtenue à l'étape précédente, en partie ou en au moins deux parties distinctes ;
- de fabrication du dispositif médical par méthode additive ; et
- d'intégration dans le dispositif médical du système de fermeture.

Avantageusement, l'étape de génération d'un maillage final en appliquant différents modificateurs comprend en outre les étapes suivantes :
- de transformation des polygones contenus dans le maillage obtenu à l'étape de génération du maillage basse résolution ;
- de génération de la structure du dispositif médical ;
- de redimensionnement de la structure du dispositif médical ;
- de lissage de la structure du dispositif médical ; et
- de nettoyage de la structure du dispositif médical.

De façon avantageuse, le procédé comprend en outre une étape de génération et d'exportation du fichier contenant le modèle 3D du dispositif médical à fabriquer. Selon une caractéristique particulière, le procédé comprend en outre une étape de définition des instructions de fabrication du dispositif médical à fabriquer.

Selon une autre caractéristique particulière le procédé comprend en outre une étape de nettoyage du dispositif médical qui a été fabriqué.

Ainsi, cette invention permet d'obtenir un dispositif médical ayant une efficacité de fixation ainsi qu'un bon maintien des au moins deux demi-coques. Par ailleurs, le fait d'utiliser des aimants rend le système de fermeture discret par rapport à d'autres solutions existantes comme celles par joints toriques, ou fermeture autoagrippante. De plus, le fait que le système de fermeture soit intégré à la structure, et qu'aucun n'élément dudit système ne se trouve sur la périphérie de la structure évite tout arrachement et accroche avec des objets présents dans l'environnement proche de l'utilisateur.

Avantageusement, et grâce au système de fermeture intégré, il est également possible de recouvrir plus facilement ce dispositif médical par un vêtement, car le volume dudit dispositif est réduit.

Les concepts fondamentaux de l'invention venant d'être exposés ci-dessus dans leur forme la plus élémentaire, d'autres détails et caractéristiques ressortiront plus clairement à la lecture de la description qui suit et en regard des dessins annexés.

### BRÈVE DESCRIPTION DES FIGURES

Les figures sont données à titre purement illustratif pour une meilleure compréhension de l'invention sans en limiter la portée. Les différents éléments peuvent être représentés de manière schématique et ne sont pas nécessairement à la même échelle. Sur l'ensemble des figures, les éléments identiques ou équivalents portent la même référence numérique.

Il est ainsi illustré en :
- Figure 1A : une vue éclatée et en perspective d'un dispositif médical ouvert de maintien d'un membre ou d'une articulation selon un premier mode de réalisation de l'invention ;
- Figure 1B : une vue en perspective du dispositif médical fermé de maintien d'un membre ou d'une articulation selon le premier mode de réalisation de l'invention ;
- Figure 2A : une vue éclatée et en perspective d'un dispositif médical ouvert de maintien d'un membre ou d'une articulation selon un second mode de réalisation de l'invention ;
- Figure 2B : une vue en perspective du dispositif médical fermé de maintien d'un membre ou d'une articulation selon le second mode de réalisation de l'invention ;
- Figure 3A : une vue éclatée et en perspective d'un dispositif médical ouvert de maintien d'un membre ou d'une articulation selon un troisième mode de réalisation de l'invention ;
- Figure 3B : une vue en perspective du dispositif médical fermé de maintien d'un membre ou d'une articulation selon le troisième mode de réalisation de l'invention ;
- Figure 4A : une vue éclatée et en perspective d'un dispositif médical ouvert de maintien d'un membre ou d'une articulation selon un quatrième mode de réalisation de l'invention ;
- Figure 4B : une vue en perspective du dispositif médical fermé de maintien d'un membre ou d'une articulation selon le quatrième mode de réalisation de l'invention ; et
- Figure 5 : les principales étapes d'un procédé de fabrication du dispositif médical, selon un mode de réalisation de l'invention.

### DESCRIPTION DÉTAILLÉE DE MODES DE RÉALISATION

Il convient de noter que certains éléments techniques bien connus de l'homme du métier sont ici décrits pour éviter toute insuffisance ou ambiguïté dans la compréhension de la présente invention.

Dans le mode de réalisation décrit ci-après, on fait référence à un dispositif médical pour poignet, sans que cela ne présente une limite.

En effet, l'invention s'adapte parfaitement aux coudes, genoux, chevilles notamment.

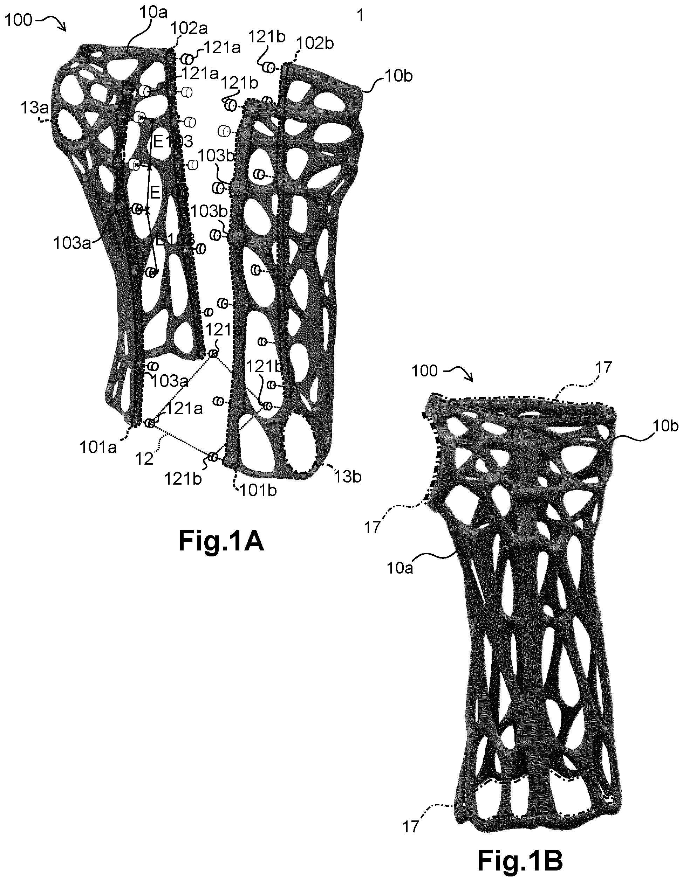

La figure 1A représente une vue éclatée et en perspective d'un dispositif médical 100 pour maintenir un membre selon un premier mode de réalisation de l'invention. Le dispositif médical 100 comporte principalement une première demi-coque 10a et une deuxième demi-coque 10b, la première demi-coque 10a et la deuxième demi-coque 10b étant complémentaires afin de former une fois assemblée une attelle ou une orthèse.

Les deux demis-coques 10a et 10b comportent une pluralité d'alvéoles 13a-b conférant auxdites demis-coques une forme sensiblement alvéolaire afin de ne pas couvrir entière la surface de la peau au niveau du dispositif médical 100. Ainsi, le membre ou l'articulation immobilisé par le dispositif médical 100 présente une aération optimale de la peau, en comparaison avec les dispositifs existants, tout en présentant une rigidité suffisante pour l'immobilisation, en optimisation la forme des alvéoles 13a-b ainsi que leurs positions sur ledit dispositif. par ailleurs, cette optimisation minimise également la quantité de matière utilisée pour fabriquer le dispositif médical 100, ce qui représente un avantage économique mais aussi environnemental.

La première demi-coque 10a et la deuxième demi-coque 10b s'assemblent au moyen d'un système de fermeture 12 qui comportent une pluralité d'aimant 121a-b allant par paire.

Dans ce mode de réalisation particulier, la demi-coque 10a comporte deux surfaces de contact 101a et 102a, ces deux surfaces de contact étant complémentaires avec deux surfaces de contact 101b et 102b de la demi-coque 10b.

Ainsi, la surface de contact 101a est complémentaire à la surface de contact 101b, et la surface de contact 102a est complémentaire à la surface de contact 102b. Dans le mode de réalisation préféré, les surfaces de contact 101a-b et 102a-b sont planes, ou sensiblement planes.

Dans d'autres modes de réalisation, les surfaces de contact 101a-b et 102a-b sont courbes tout en conservant leurs propriétés de complémentarité, et tel que décrit précédemment.

Préférablement, les aimants 121a-b sont collés dans des logements 103a-b situés sur les surfaces de contact 101a-b et 102a-b des demis-coques 10a-b.

Le système de fermeture 12 est donc réalisé par la force d'attraction des aimants 121a-b.

Dans un mode de réalisation particulier, les aimants 121a-b sont répartis uniformément le long des surfaces de contact 101a-b et 102a-b, en étant espacé d'une distance E103.

Dans un mode de réalisation particulier, les aimants 121a-b sont répartis de manière non uniforme le long des surfaces de contact 101a-b et 102a-b afin notamment de prendre en compte les variations de contraintes mécaniques hétérogènes dans le dispositif médical 100. Ainsi, entre deux aimants consécutifs 121a d'une même surface de contact 101a-b, ou entre deux aimants consécutifs 121b d'une même surface de contact 102a-b, la distance E103 est adaptée au cas par cas. Préférablement, les aimants 121a-b ont une force d'adhérence qui est sensiblement identique.

Dans un autre mode de réalisation, les aimants 121a-b ont une force d'adhérence qui est fonction de l'emplacement desdits aimants. Ainsi, et pour une zone du dispositif médical 100 qui présente des risques de désolidarisation, par exemple au niveau d'une articulation ou au niveau des extrémités dudit dispositif, les aimants 121a-b ont une force d'adhérence qui est plus élevée, en comparaison avec ceux d'une zone où ces risques sont faibles, par exemple éloigné d'une articulation ou dans une partie centrale dudit dispositif. Le système de fermeture 12 est alors optimisé en fonction des risques d'ouverture ainsi que des contraintes mécaniques qui sont appliquées sur le dispositif médical 100.

Avantageusement, les demis-coques 10a-b étant fabriquées au moyen d'un procédé de fabrication additive, les logements 103a-b sont créés sans perçage par outillage desdites surfaces, mais directement conçus dans le modèle 3D numérique du dispositif médical 100.

Le système de fermeture 12 est donc parfaitement invisible une fois les deux demis-coques 10a-b assemblées, améliorant l'aspect esthétique du dispositif médical 100 et limitant les risques que ledit dispositif ne s'accroche avec des éléments présents dans l'environnement proche du patient. Par ailleurs, le système de fermeture 12 étant intégralement intégré au sein de la structure du dispositif médical 100, aucun élément ne dépasse des demis-coques 10a-b, conférant audit dispositif une faible épaisseur, et facilitant notamment le recouvrement dudit dispositif par un vêtement, même si celui-ci serait porté proche du corps.

Avantageusement, les aimants 121a-b assurent un excellent maintien entre les deux demis-coques, et donc une bonne contention du membre traité.

Dans un mode de réalisation alternatif, non représenté ici, le système de fermeture 12 comporte une pluralité de paires « aimant-cylindre métallique », notamment pour réduire le coût de production du dispositif médical 100, un seul aimant, associé à un support métallique dans ladite paire étant nécessaire pour assurer l'assemblage dudit dispositif.

Par ailleurs, pour désolidariser les deux demis-coques 10a-b, il est nécessaire d'appliquer une force de traction supérieure à plusieurs kilos. Cela assure également une sécurité à l'utilisation et lors des mouvements du patient, le dispositif médical 100 ne pouvant pas se désassembler facilement.

Les deux demis-coques 10a-b s'installent facilement autour du membre ou de l'articulation à maintenir car le système de fermeture 12 est automatique, en comparaison avec d'autres dispositifs médicaux de l'art antérieur tels que par joints toriques, bande adhésives ou fermetures autoagrippantes.

Avantageusement, et grâce au magnétisme du système de fermeture 12, le guidage et l'assemblage des deux demis-coques 10a-b est réalisé avec facilité et sans qu'il soit nécessaire d'ajouter des éléments supplémentaires au niveau des surfaces de contact tels que des trous et des tétons. Ainsi, il n'y a pas de mouvement de glissement entre les deux demis-coques 10a-b, et un patient est en capacité d'assembler par lui-même le dispositif médical 100, notamment quand il s'agit d'un des membres supérieurs qui doit être immobilisé, renforçant ainsi l'autonomie du patient.

La figure 1B représente vue en perspective du dispositif médical 100 lorsqu'il est complètement assemblé. Une fois assemblé, le dispositif médical 100 présente également des orifices de passage 17 pour le passage de membres ou d'articulations. Dans le mode de réalisation présenté à la figure 1B, le dispositif médical 100 comporte trois orifices de passage 17 pour y loger le pouce, le reste des doigts le reste du membre bras d'un patient.

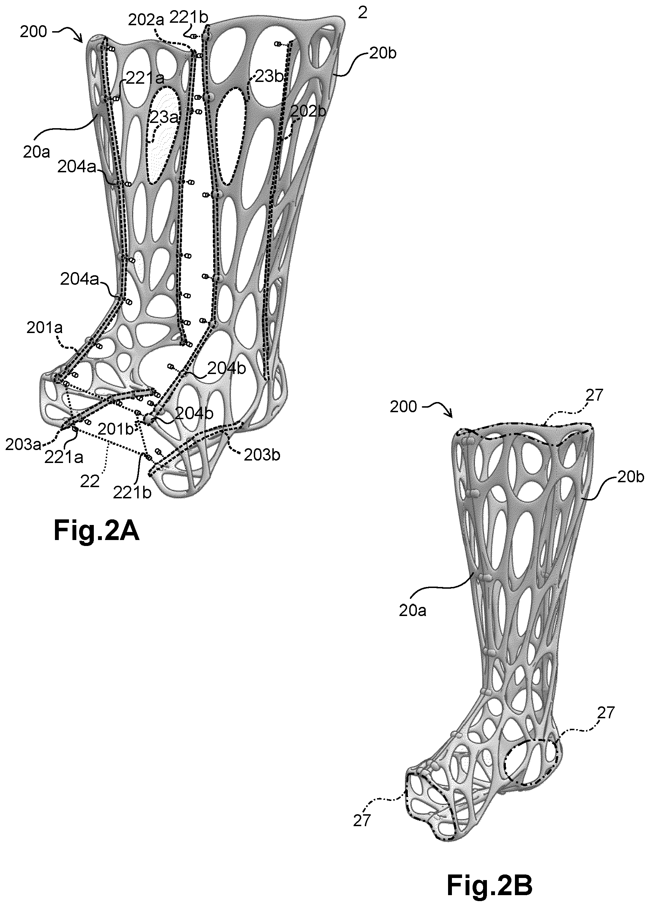

La figure 2A représente une vue éclatée et en perspective d'un dispositif médical 200 selon un deuxième mode de réalisation de l'invention, et pour être utilisé autour d'une jambe.

Comme précédemment, le dispositif médical 200 comporte principalement une première demi-coque 20a et une deuxième demi-coque 20b, la première demi-coque 20a et la deuxième demi-coque 20b étant complémentaires afin de former une fois assemblée une attelle ou une orthèse, comme cela est visible sur la figure 2B.

La première demi-coque 20a et la deuxième demi-coque 20b s'assemblent au moyen d'un système de fermeture 22, qui dans ce mode de réalisation, comportent une pluralité d'aimant 121a-b allant par paire. De plus, la première demi-coque 20a comporte ici trois surfaces de contact 201a, 202a et 203a, ces trois surfaces de contact étant complémentaires avec trois surfaces de contact 201b, 202b et 203b de la seconde demi-coque 20b. Les surfaces de contact 201a-b, 202a-b et 203a-b sont planes, ou sensiblement planes. Dans d'autres modes de réalisation, les surfaces de contact 201a-b, 202a-b et 203a-b sont courbes tout en conservant leurs propriétés de complémentarité, et tel que décrit précédemment.

Préférablement, les aimants 221a-b sont collés dans des logements 204a-b situés sur les surfaces de contact 201a-b, 202a-b et 203a-b, lesdits logements 204a-b étant créés sans perçage par outillage desdites surfaces, mais directement conçus dans le modèle 3D numérique du dispositif médical 200.

De nouveau, le système de fermeture 22 est donc parfaitement invisible une fois les deux demis-coques 20a-b assemblées, et présente les mêmes avantages listés précédemment.

De plus, les deux demis-coques 20a et 20b comportent également une pluralité d'alvéoles 23a-b.

La figure 2B représente quant à elle vue en perspective du dispositif médical 200 lorsqu'il est complètement assemblé. Comme il est possible de le constater sur la figure 2B, le dispositif médical 200 présente également des orifices de passage 27 pour le passage de membres ou d'articulations. Dans le mode de réalisation présenté à la figure 2B, le dispositif médical 200 comporte trois orifices de passage 27 afin d'y loger le talon, la jambe et l'avant-pied d'un patient.

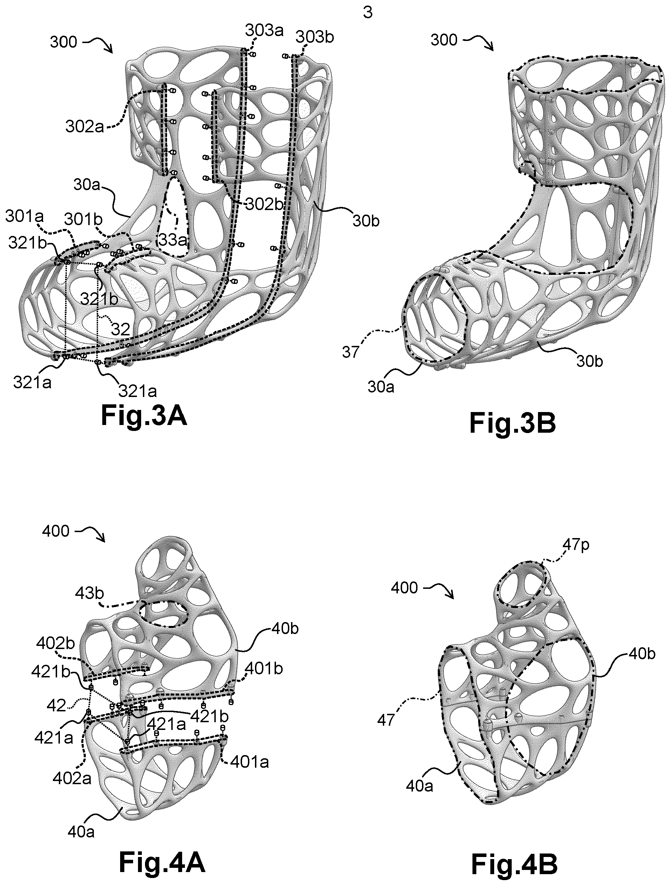

La figure 3A représente une vue éclatée et en perspective d'un dispositif médical 300 pour immobiliser un coude selon un troisième mode de réalisation de l'invention.

Le dispositif médical 300 comportent également une première demi-coque 30a et une deuxième demi-coque 30b qui sont complémentaires afin de former une fois assemblées une attelle ou une orthèse, comme cela est visible sur la figure 3B. Comme précédemment, la première demi-coque 30a et la deuxième demi-coque 30b s'assemblent au moyen d'un système de fermeture 32, qui dans ce mode de réalisation, comportent une pluralité d'aimant 321a-b allant par paire. Dans ce mode de réalisation, la première demi-coque 30a comporte ici trois surfaces de contact 301a, 302a et 303a, ces trois surfaces de contact étant complémentaires avec trois surfaces de contact 301b, 302b et 303b de la seconde demi-coque 30b.

Les aimants 321a-b sont préférablement collés dans des logements 304a-b situés sur les surfaces de contact 301a-b, 302a-b et 303a-b, lesdits logements 304a-b étant créés sans perçage par outillage desdites surfaces, mais directement conçus dans le modèle 3D numérique du dispositif médical 300.

De nouveau, le système de fermeture 32 est donc parfaitement invisible une fois les deux demis-coques 30a-b assemblées, et présentent les mêmes avantages listés précédemment.

Les deux demis-coques 30a et 30b comportent comme pour les modes de réalisation précédents une pluralité d'alvéoles 33a-b.

La figure 3B représente quant à elle vue en perspective du dispositif médical 300 lorsqu'il est complètement assemblé. Comme il est possible de le constater sur la figure 3B, le dispositif médical 300 présente également des orifices de passage 37 pour le passage de membres ou d'articulations. Dans le mode de réalisation présenté à la figure 3B, le dispositif médical 300 comporte trois orifices de passage 37 afin d'y loger le coude, le bras et la main d'un patient.

La figure 4A représente une vue éclatée et en perspective d'un dispositif médical 400 pour immobiliser une main selon un quatrième mode de réalisation de l'invention.

Le dispositif médical 400 comportent une première demi-coque 40a et une deuxième demi-coque 40b qui sont complémentaires afin de former une fois assemblées une attelle ou une orthèse, comme cela est visible sur la figure 4B. Comme précédemment, la première demi-coque 40a et la deuxième demi-coque 40b s'assemblent au moyen d'un système de fermeture 42, qui dans ce mode de réalisation, comportent une pluralité d'aimant 421a-b allant par paire. Dans ce mode de réalisation, la première demi-coque 40a comporte ici trois surfaces de contact 401a, 402a et 403a, ces trois surfaces de contact étant complémentaires avec trois surfaces de contact 401b, 402b et 403b de la seconde demi-coque 40b.

Les aimants 421a-b sont préférablement collés dans des logements 404a-b situés sur les surfaces de contact 401a-b, 402a-b et 403a-b, lesdits logements 404a-b étant créés sans perçage par outillage desdites surfaces, mais directement conçus dans le modèle 3D numérique du dispositif médical 400.

De nouveau, le système de fermeture 42 est donc parfaitement invisible une fois les deux demis-coques 40a-b assemblées, et présentent les mêmes avantages listés précédemment.

Les deux demis-coques 40a et 40b comportent comme pour les modes de réalisation précédents une pluralité d'alvéoles 43a-b.

La figure 4B représente quant à elle vue en perspective du dispositif médical 400 lorsqu'il est complètement assemblé. Comme il est possible de le constater sur la figure 4B, le dispositif médical 400 présente également des orifices de passage 47 pour le passage de membres ou d'articulations. Dans le mode de réalisation présenté à la figure 4B, le dispositif médical 400 comporte trois orifices de passage 47 afin d'y loger le pouce, la paume de main, et les doigts d'un patient. Les orifices de passage 47 sont, soit formés par l'assemblage des deux demis-coques 40a et 40b, soit conçus dans le modèle 3D numérique du dispositif médical 400, comme c'est le cas ici pour un orifice de passage 47p destiné à y loger un pouce.

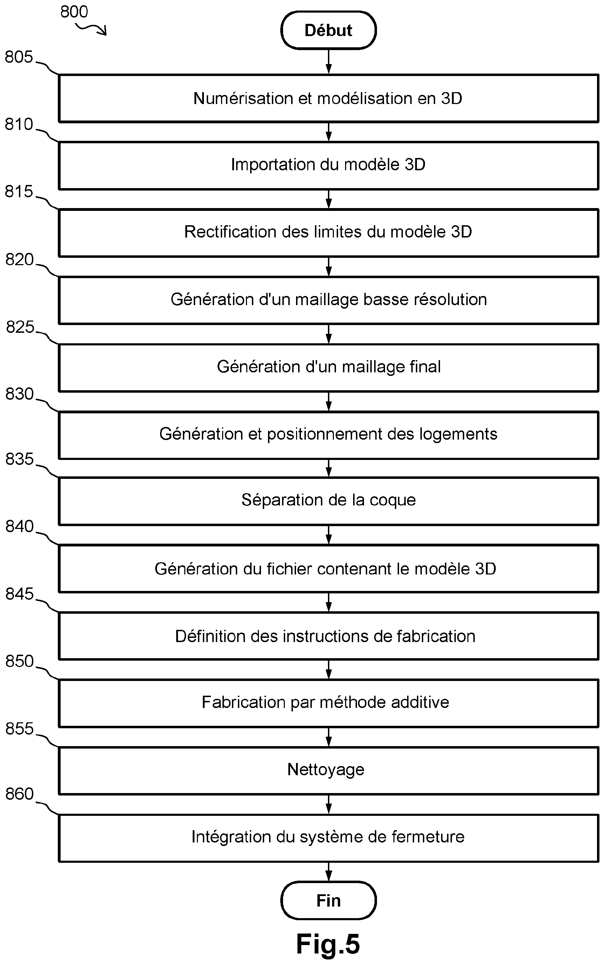

La figure 5 représente les étapes d'un procédé de fabrication 800 du dispositif médical selon un mode de réalisation de l'invention.

Le procédé de fabrication 800 comprend principalement :
- une étape 805 de numérisation et de modélisation en trois dimensions (3D) pour obtenir un modèle 3D d'un membre ou d'une l'articulation à immobiliser ;
- une étape 810 d'importation du modèle 3D dans le logiciel de conception assistée par ordinateur (CAO) et de centrage du modèle 3D du membre ou de l'articulation à immobiliser dans un référentiel du logiciel de CAO ;
- une étape 815 de rectificatif de limites du modèle 3D du membre ou de l'articulation à immobiliser ;
- une étape 820 de génération d'un premier maillage, dît maillage « basse résolution » du modèle 3D obtenu à l'étape 815 ;
- une étape 825 de génération d'un second maillage, dît maillage « final » en appliquant différents modificateurs sur le maillage basse résolution obtenu à l'étape précédente, ledit maillage définissant les demis-coques 10a-b, 20a-b, 30a-b et 40a-b du dispositif médical 100, 200, 300 ou 400 ;
- une étape 830 de génération et de positionnement des logements 104a-b - 404a-b pour les aimants dans les emplacements prévus à ces effets;
- une étape 835 de séparation de la structure du dispositif médical 100, 200, 300 ou 400, en partie ou en au moins deux parties distinctes ;
- une étape 840 de génération et d'exportation du fichier contenant le modèle 3D du dispositif médical 100-400 à fabriquer ;
- une étape 845 de définition des instructions de fabrication du dispositif médical 100-400 à fabriquer ;
- une étape 850 de fabrication du dispositif médical 100-400 par méthode additive ;
- une étape 855 de nettoyage du dispositif médical 100-400 qui a été fabriqué ;
   et
- une étape 860 d'intégration dans le dispositif médical 100-400 du système de fermeture 12-42.

Chacune des étapes qui viennent d'être listées sont décrites en détail ci-après. L'étape 805 de numérisation et de modélisation en trois dimensions du membre ou de l'articulation à immobiliser consiste à obtenir une représentation tridimensionnelle précise du membre ou de l'articulation du patient. À l'aide de techniques de numérisation avancées telles que la photogrammétrie ou la numérisation laser, des données détaillées sont capturées. Ces données sont ensuite utilisées pour créer un modèle 3D, dît « modèle haute résolution » du membre ou de l'articulation à immobiliser, offrant une base numérique précise pour la conception du dispositif médical 100-400.

L'étape 810 d'importation du modèle 3D dans le logiciel de CAO et de centrage du modèle 3D du membre ou de l'articulation à immobiliser dans un référentiel du logiciel de CAO est réalisée au moyen de logiciels CAO du commerce. Lors de cette étape, le modèle 3D obtenu avec l'étape 805 est importé puis soigneusement orienté dans le référentiel du logiciel CAO, en particulier et suivant un plan dît « plan de coupe » qui est utilisé lors d'une étape ultérieure.

L'étape 815 de rectificatif de limites du modèle 3D du membre ou de l'articulation à immobiliser consiste à définir les différentes limites du dispositif médical 100, 200, 300 ou 400, lesdites limites correspondant aux orifices de passage, 17, 27, 37, 47 et 47p sur le modèle 3D. Pour ce faire le modèle 3D importé est rogné des parties du corps qui ne nécessité pas d'être recouvertes par le dispositif médical 100, 200, 300 ou 400, telles que, et en fonction du membre ou de l'articulation à immobiliser, les doigts, les orteils. Ainsi, et à l'issue de l'étape 815 de rectification, le modèle 3D obtenu représente la surface de la peau qui sera recouverte par le dispositif médical 100, 200, 300 ou 400.

L'étape 820 de génération d'un premier maillage, dît « maillage basse résolution » du modèle 3D obtenu à l'étape 815 consiste à transformer le modèle 3D obtenu à l'étape précédente, qui est en haute résolution, en un maillage basse résolution de cette même forme. Dans un mode de réalisation particulier, il en résulte un maillage constitué de faces carrées pouvant comprendre entre 120 et 160 faces. Les faces du maillage peuvent être en d'autres formes polynomiales, le maillage comportant une unique forme de face, ou une combinaison d'au moins deux types de formes de faces. Lors de cette même étape, une étape 8200 de nettoyage du maillage basse résolution (non représentée sur la figure) est réalisée et consiste à supprimer des faces aberrantes ou inutiles, ainsi qu'à fusionner une pluralité de faces pour obtenir des faces ayant une plus grande superficie. Ces nouvelles faces correspondent aux alvéoles 13a-b, 23a-b, 33a-b et 43a-b présentées aux figures précédentes.

L'étape 825 de génération du second maillage, dît « maillage final » est ensuite réalisée en appliquant différents modificateurs sur le maillage basse résolution obtenu à l'étape précédente. Les modificateurs sont des opérations mathématiques qui sont disponibles dans le logiciel CAO et qui sont utilisés pour obtenir la forme finale du dispositif médical 100, 200, 300 ou 400.

Dans un mode de réalisation non représenté à la figure 5, l'étape 825 de génération du second maillage comprend successivement :
- une étape 8250 de transformation des polygones contenus dans le maillage obtenu à l'étape 820 de génération du maillage basse résolution ;
- une étape 8251 de génération de la structure du dispositif médical 100, 200, 300 ou 400 ;
- une étape 8252 de redimensionnement de la structure du dispositif médical 100, 200, 300 ou 400 ;
- une étape 8253 de lissage de la structure du dispositif médical 100, 200, 300 ou 400 ;
- une étape 8254 de nettoyage de la structure du dispositif médical 100, 200, 300 ou 400.

Chacune des étapes qui viennent d'être listées sont décrites en détail ci-après. L'étape 8250 de transformation des polygones contenus dans le maillage obtenu à l'étape 820 de génération du maillage basse résolution consiste à modifier la forme géométrique des faces du maillage, en passant de quadrilatères convexes à des polygones convexes constitués d'au moins cinq côtés, généralement des pentagones et/ou des hexagones.

L'étape 8251 de génération de la structure du dispositif médical 100, 200, 300 ou 400 est ensuite réalisée. Par structure, il est entendu la partie solide du dispositif médical 100, 200, 300 ou 400 et qui sera imprimée ultérieurement. A partir du maillage du modèle 3D obtenu précédemment, ainsi que d'au moins un paramètre tel que un diamètre de la structure, une épaisseur de la structure, ladite structure est automatiquement générée. Lors de cette étape, les alvéoles 13a-b, 23a-b, 33a-b et 43a-b représentées aux figures 1 è 4 sont créées. Pour rappel, en comparaison avec les dispositifs médicaux pleins, les alvéoles 13a-b, 23a-b, 33a-b et 43a-b permettent d'améliorer la ventilation du membre ou de l'articulation, favorisant ainsi le confort du patient.

Ensuite, l'étape 8252 de redimensionnement de la structure du dispositif médical 100, 200, 300 ou 400 est réalisée. Cette étape consiste à modifier les dimensions de la structure pour que ladite structure épouse la surface de la peau qui est matérialisée par le modèle 3D haute résolution importé à l'étape 810 du procédé 800. La structure est soigneusement ajustée pour envelopper le membre ou l'articulation de manière ergonomique, prenant en compte les besoins médicaux spécifiques du patient. Dans certains modes de réalisation, la conception de la structure tient également compte des points de pression, des mouvements naturels du membre et des critères médicaux requis pour garantir un ajustement optimal sur le membre ou l'articulation du patient.

Ensuite, l'étape 8253 de lissage de la structure du dispositif médical 100, 200, 300 ou 400 est réalisée pour que ledit dispositif présente peu de rugosité et soit confortable à porter.

Enfin, l'étape 8254 de nettoyage de la structure du dispositif médical 100, 200, 300 ou 400 consiste à supprimer des éléments jugés inutiles par l'orthoprothésiste, tels que des alvéoles 13a-b, 23a-b, 33a-b ou 43a-b.

A l'issue des étapes décrites précédemment, la structure du dispositif médical 100, 200, 300 ou 400 qui est obtenue constitue la structure dans sa forme quasi-définitive, et les étapes suivantes vont finaliser

L'étape 830 de génération et de positionnement du système de fermeture 12, 22, 32 ou 42 sur la structure du dispositif médical 100, 200, 300 ou 400 est ensuite réalisée. Lors de cette étape, le positionnement des logements 104a-b, 204a-b, 304a-b ou 404a-b est réalisé par rapport au plan de coupe qui a été utilisé lors de l' étape 810 d'importation du modèle 3D pour aligner ledit modèle. De plus, les logements 104a-b, 204a-b, 304a-b ou 404a-b y sont positionnés perpendiculairement par rapport au plan de coupe. Les logements 104a-b, 204a-b, 304a-b ou 404a-b sont soigneusement positionnés et dimensionnés pour assurer un maintien sécurisé et confortable du dispositif médical 100, 200, 300 ou 400. En outre, une opération booléenne est appliquée pour créer les logements 104a-b, 204a-b, 304a-b ou 404a-b dans la structure du dispositif médical 100, 200, 300 ou 400 afin d'y accueillir les aimants 121a-b, 221a-b, 321a-b ou 421a-b.

Pour finir la phase de conception numérique du dispositif médical 100, 200, 300 ou 400, l'étape 835 de séparation de la structure, en partie ou en au moins deux parties, consiste à créer une séparation partielle ou complète de ladite structure, en tenant compte de la géométrie du membre ou de l'articulation. Cette séparation partielle ou complète fait apparaître les surfaces de contacts sur lesquelles les systèmes de fermeture 12a-b, 22a-b, 32a-b et 42a-b sont intégrés ultérieurement. Cette séparation présente plusieurs avantages. Tout d'abord, le fait que le dispositif médical 100-400 soit en plusieurs parties a pour but de faciliter son assemblage sur le patient. Aussi, cela facilite la fabrication du dispositif médical 100-400 au moyen de la méthode par fabrication additive.

L'étape 840 de génération du fichier contenant le modèle 3D du dispositif médical 100-400 à fabriquer consiste à générer le fichier contenant l'ensemble des modifications apportées lors des étapes précédentes ,et donc d'un modèle 3D dans sa version finale avant fabrication. Le format du fichier contenant le modèle 3D est par exemple du type STL, OBJ, AMF ou 3MF, sans que cela ne soit une limite à la présente invention.

L'étape 845 de définition des instructions de fabrication du dispositif médical 100-400 à fabriquer consiste à importer le fichier du modèle 3D obtenu à l'étape précédente dans un logiciel de découpage de modèles 3D (plus communément appelé par sa terminologie anglaise « *slicer* »), puis de générer un fichier contenant les instructions de fabrication du dispositif médical 100-400. Ces instructions détaillées incluent les paramètres d'impression, le type de matériau, et l'orientation optimale pour obtenir le meilleur résultat lors de la fabrication additive.

Dans un autre mode de réalisation de l'invention, l'étape 840 de génération du fichier contenant le modèle 3D et l'étape 845 de définition des instructions de fabrication du dispositif médical 100-400 à fabriquer sont faites lors d'une seule et même étape.

L'étape 850 de fabrication du dispositif médical 100-400 par méthode additive consiste en la phase d'impression 3D dudit dispositif. Des technologies adaptées telles que la stéréolithographie ou le dépôt de fil fondu sont utilisées en fonction des exigences spécifiques du matériau et du design. L'étape 850 transforme le modèle 3D en un produit physique.

L'étape 855 de nettoyage du dispositif médical 100-400 qui a été fabriqué consiste notamment à éliminer les éventuels supports d'impression qui ont été utilisés, les résidus ainsi que les aspérités de surface. L'étape 855 assure un produit final propre, lisse et prêt à être utilisé pour immobiliser un membre ou une articulation. A des fins de finitions, le dispositif médical 100-400 peut être poncé et/ou un produit de lissage à froid peut être utilisé afin d'améliorer l'état de surface dudit dispositif. L'étape 860 d'intégration dans le dispositif médical du système de fermeture 12-42 consiste à intégrer dans les logements 104a-b - 404a-b les aimants. L'étape 860 permet de compléter le dispositif médical 100-400, afin qu'il soit prêt à être fourni au patient.

Dans certains modes de réalisations, toute ou partie des étapes décrite précédemment peuvent être réalisées manuellement ou de façon automatisée, par exemple au moyen d'algorithmes d'optimisation.

Au final, et en comparaison avec les dispositifs médicaux connus, le dispositif médical 100, 200, 300 et 400 permet une utilisation aisée et confortable par le patient, tout en favorisant son autonomie lors de l'installation ainsi que l'adhésion thérapeutique dudit dispositif.

## Revendications

1. Dispositif médical (100,200,300,400) pour immobiliser un membre ou une articulation, ledit dispositif comportant au moins deux demis-coques complémentaires (10a-b,20a-b,30a-b,40a-b) de forme sensiblement alvéolaire, lesdites demis-coques comprenant chacune au moins deux surfaces de contact (101a-b,102a-b,201a-b,202a-b,203a-b,301a-b,302a-b,303a-b,401a-b,402a-b), un système de fermeture (12,22,32,42), le dispositif étant **caractérisé en ce que** le système de fermeture (12,22,32,42) est positionné dans des logements (103a-b,204a-b,304a-b,403a-b) des surfaces de contact (101a-b,102a-b,201a-b,202a-b,203a-b,301a-b,302a-b,303a-b,401a-b,402a-b), ledit système de fermeture étant magnétique, ledit système de fermeture étant invisible lorsque le dispositif médical (100,200,300,400) est assemblé.

2. Dispositif médical (100,200,300,400) selon la revendication 1, dans lequel le système de fermeture comporte une pluralité d'aimant (121a-b,221a-b, 321a-b,421a-b), chaque aimant (121a-b,221a-b, 321a-b,421a-b) étant positionné un seul à la fois dans le logement (103a-b,204a-b,304a-b,403a-b) qui lui est dédié.

3. Dispositif médical (100,200,300,400) selon la revendication 1, dans lequel le système de fermeture comporte une pluralité de paires « aimant-cylindre métallique ».

4. Dispositif médical (100) selon l'une quelconque des revendications précédentes, dans lequel les logements (103a-b,204a-b,304a-b,403a-b) du système de fermeture (12,22,32,42) sont disposés régulièrement sur les surfaces de contact (101a-b,102a-b,201a-b,202a-b,203a-b,301a-b,302a-b,303a-b,401a-b,402a-b).

5. Dispositif médical (100) selon l'une quelconque des revendications 1 à 3, dans lequel les logements (103a-b,204a-b,304a-b,403a-b) du système de fermeture (12,22,32,42) sont disposés irrégulièrement sur les surfaces de contact (101a-b, 102a-b,201a-b,202a-b,203a-b,301a-b,302a-b,303a-b,401a-b,402a-b).

6. Dispositif médical (100) selon l'une quelconque des revendications 1 à 3, dans lequel les logements (103a-b,204a-b,304a-b,403a-b) du système de fermeture (12,22,32,42) sont disposés sur les surfaces de contact (101a-b,102a-b,201a-b,202a-b,203a-b,301a-b,302a-b,303a-b,401a-b,402a-b) proportionnellement par rapport aux efforts mécaniques qui sont exercés sur ledit dispositif.

7. Dispositif médical (100) selon l'une quelconque des revendications précédentes, dans lequel les aimants (121a-b,221a-b, 321a-b,421a-b) ont une adhérence magnétique identique.

8. Dispositif médical (100) selon l'une quelconque des revendications précédentes, dans lequel les aimants (121a-b,221a-b, 321a-b,421a-b) ont une adhérence magnétique différente.

9. Dispositif médical (100) selon la revendication 6, l'adhérence magnétique des aimants (121a-b,221a-b, 321a-b,421a-b) tient compte des efforts de traction qui sont exercés au niveau des emplacement desdits aimants sur ledit dispositif.

10. Procédé (800) de fabrication d'un dispositif médical (100,200,300,400) selon l'une quelconque des revendications précédentes, **caractérisé en ce qu'**il comprend les étapes suivantes :
- (805) de numérisation et de modélisation en trois dimensions (3D) pour obtenir un modèle 3D d'un membre ou d'une l'articulation à immobiliser ;
- (810) d'importation du modèle 3D dans le logiciel de conception assistée par ordinateur (CAO) et de centrage du modèle 3D du membre ou de l'articulation à immobiliser dans un référentiel du logiciel de CAO ;
- (815) de rectificatif de limites du modèle 3D du membre ou de l'articulation à immobiliser ;
- (820) de génération d'un maillage basse résolution du modèle 3D obtenu à l'étape précédente ;
- (825) de génération d'un maillage final en appliquant différents modificateurs sur le maillage basse résolution obtenu à l'étape précédente ;
- (830) de génération et de positionnement des logements (104a-b, 204a-b, 304a-b ou 404a-b) ;
- (835) de séparation d'une structure du dispositif médical (100, 200, 300, 400) obtenue à l'étape précédente, en partie ou en au moins deux parties distinctes ;
- (850) de fabrication du dispositif médical (100, 200, 300, 400) par méthode additive ; et
- (860) d'intégration dans le dispositif médical (100, 200, 300, 400) du système de fermeture (12, 22, 32 42).

11. Procédé (800) selon la revendication 10, l'étape (825) de génération d'un maillage final en appliquant différents modificateurs comprenant en outre les étapes suivantes :
- (8250) de transformation des polygones contenus dans le maillage obtenu à l'étape (820) de génération du maillage basse résolution ;
- (8251) de génération de la structure du dispositif médical (100, 200, 300, 400) ;
- (8252) de redimensionnement de la structure du dispositif médical (100, 200, 300, 400) ;
- (8253) de lissage de la structure du dispositif médical (100, 200, 300, 400) ; et
- (8254) de nettoyage de la structure du dispositif médical (100, 200, 300 ou 400).

12. Procédé (800) selon la revendication 10 ou la revendication 11, comprenant en outre une étape (840) de génération et d'exportation du fichier contenant le modèle 3D du dispositif médical (100, 200, 300, 400) à fabriquer.

13. Procédé (800) selon l'une quelconque des revendications 10 à 12, comprenant en outre une étape (845) de définition des instructions de fabrication du dispositif médical (100, 200, 300, 400) à fabriquer.

14. Procédé (800) selon l'une quelconque des revendications 10 à 13, comprenant en outre une étape (855) de nettoyage du dispositif médical (100, 200, 300, 400) qui a été fabriqué.
